# EUROPÄISCHE PATENTSCHRIFT

(11) **EP 0 789 013 B1**
(45) Veröffentlichungstag und Bekanntmachung des Hinweises auf die Patenterteilung: **26.04.2000**
(21) Anmeldenummer: 96120044.1
(22) Anmeldetag: 13.12.1996
(51) Int. Cl.: C07C 17/16, C07C 19/01

(54) **Verfahren zur Herstellung von Alkylchloriden**
Process for the preparation of alkyl chlorides
Procédé pour la préparation de chlorures d'alkyle

(30) Priorität: 08.02.1996 DE 19604567
(43) Veröffentlichungstag der Anmeldung: 13.08.1997
(73) Patentinhaber: Degussa-Hüls Aktiengesellschaft, 60287 Frankfurt am Main (DE)
(72) Erfinder: Metz, Josef, Dr., 45770 Marl (DE); Osterholt, Clemens, 46286 Dorsten (DE); Lange, Jürgen, 45721 Haltern (DE)

(56) Entgegenhaltungen:
- DE-C- 934 701
- US-A- 3 981 938

## Beschreibung

Die Erfindung betrifft ein neues Verfahren zur Herstellung von Alkylchloriden mit 6 bis 16 C-Atomen durch Umsetzung der entsprechenden Alkohole mit Chlorwasserstoff.

Alkylchloride sind wertvolle Lösemittel. Sie werden für Friedel-Crafts-Synthesen und zur Herstellung von metallorganischen Verbindungen, Pflanzenschutzmitteln oder pharmazeutischen Produkten verwendet.

Bei der Herstellung der Alkylchloride geht man im allgemeinen von Olefinen oder von Alkoholen aus.

In DE-A-39 17 190 werden Chloralkane mit 8 bis 22 C-Atomen bei 120 bis 180 °C im Batch-Verfahren hergestellt. Myristylalkohol wird beispielsweise bei 135 bis 145 °C mit Hilfe von HCl-Gas in Tetradecylchlorid übergeführt. Während der Reaktion wird das Reaktionswasser als konzentrierte Salzsäure abdestilliert. Nach der Reaktion wird der Reaktorinhalt destillativ aufgearbeitet. Man erhält ein ca. 95%iges Produkt. Bei diesem Verfahren entstehen in einer Nebenreaktion Ether in größeren Mengen, die den folgenden Ansätzen zugegeben werden müssen.

In JP 74/034 646 wird beispielsweise n-Octylalkohol, katalysiert durch Pyridin, mit HCl-Gas bei 130 °C zu n-Octylchlorid umgesetzt. Das Produkt wird bei vermindertem Druck abdestilliert. Die Ausbeute beträgt hier nur 93,6 %, bezogen auf den eingesetzten Alkohol.

DE-C-934 701 beschreibt die Kontinuierliche Herstellung von Alkylchloriden aus den korrespondienenden Alkoholen und HCl(g), wobei die Reaktion in den Gasphase durchgeführt wird.

Nach JP 73/030 606 wird n-Octanol mit Chinolin als Katalysator in einer ersten Stufe mit 28,5%iger HCl bei 110 bis 130 °C umgesetzt. Dann wird n-Octylchlorid unter reduziertem Druck abdestilliert, worauf unreagiertes n-Octanol in einer zweiten Stufe mit HCl-Gas zur Reaktion gebracht wird. Bei diesem Verfahren ist die Nebenproduktbildung sehr hoch.

DE-A-24 35 029 beschreibt die Herstellung von Monochloralkanen bzw. -cycloalkanen mit mehr als 4 C-Atomen in einem Reaktor mit großer Oberfläche. Dabei wird eine mit HCl gesättigte wäßrige Mischung aus ZnC₂ und Alkohol von oben nach unten geleitet, wobei man gasförmiges HCl entgegenströmen läßt. Dieses Verfahren erfordert eine spezielle Apparatur sowie eine Katalysatoraufarbeitung und -rückführung.

In CZ 148 797 werden Alkylhalogenide mit 1 bis 8 C-Atomen aus den entsprechenden Alkoholen hergestellt. Dabei wird im Batch-Prozeß beispielsweise Butylchlorid mit unreagiertem Alkohol und Wasser azeotrop abdestilliert. Nach Phasentrennung, bei der die wäßrige Phase verworfen wird, wird die organische Phase zurückgeführt, bis nach mehreren Zyklen und erst nach ca. 12 Stunden Butylchlorid bei befriedigenden Umsätzen erhalten wird.

Aufgabe der vorliegenden Erfindung war es daher, ein einfaches Verfahren bereitzustellen, nach dem Alkylchloride in hohen Raum/Zeit-Ausbeuten aus Alkoholen hergestellt werden können.

Die Aufgabe wird erfindungsgemäß dadurch gelöst, daß man die Reaktion bei einer Temperatur unterhalb der Siedetemperatur des Alkylchlorids durchführt und das gebildete Alkylchlorid mit Hilfe von zusätzlich eingespeister konzentrierter Salzsäure über Kopf abdestilliert.

Nach dem Verfahren hergestellte Alkylchloride können linear oder verzweigt sein. Beispiele dafür sind n-Hexylchlorid, n-Octylchlorid, Decyl-, Dodecyl- und Tetradecylchlorid. Neben Mono- können auch Dichloride hergestellt werden. Vorzugsweise werden Alkylchloride mit 6 bis 10 C-Atomen synthetisiert.

Vorzugsweise liegt die Reaktionstemperatur 5 bis 100 °C unterhalb des Siedepunkts des gebildeten Alkylchlorids.

Durch Einleiten einer vorzugsweise erwärmten konzentrierten wäßrigen Salzsäure wird das Produkt nach einer Art Wasserdampfdestillation abgetrennt. Konzentrierte Salzsäuren im Sinne dieser Erfindung sind 20- bis 45%ige Salzsäuren. Vorzugsweise werden 30- bis 40%ige Salzsäuren verwendet.

Das Verfahren kann ansatzweise, semikontinuierlich oder kontinuierlich durchgeführt werden. Bevorzugt wird das Herstellverfahren kontinuierlich ausgeführt.

Das Verfahren liefert Alkylchloride bei hoher Raum/Zeit-Ausbeute mit verbesserter Selektivität. Eine anschließende destillative Aufarbeitung ist nicht erforderlich. Der Anfall an Reststoffen ist gering.

Die folgenden Beispiele sollen die Erfindung verdeutlichen.

### Beispiel 1

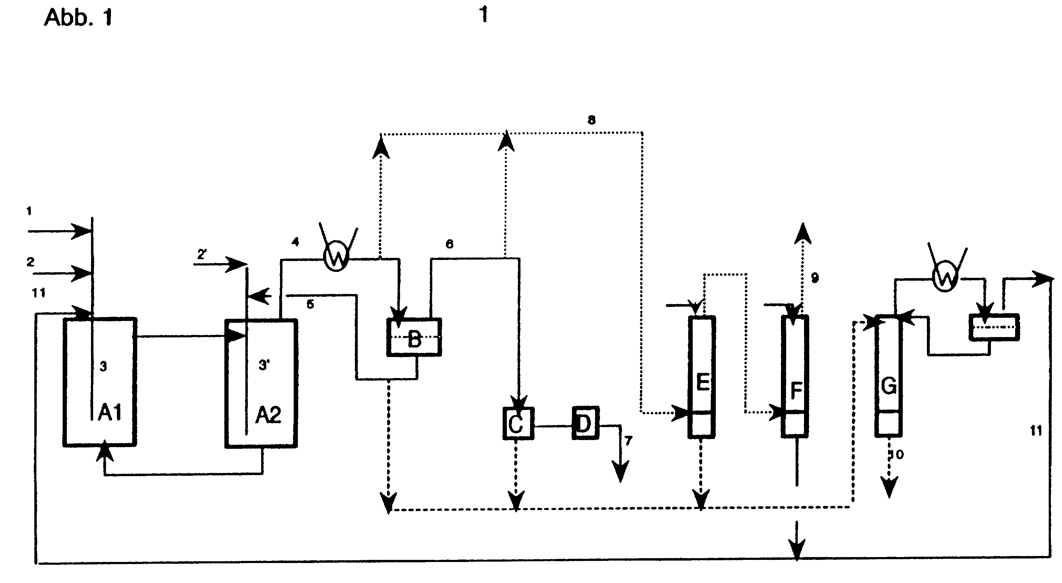

### Kontinuierliche Herstellung von n-Octylchlorid (Abb. 1)

Zwei in Reihe geschaltete 2-1- Glasreaktoren (A₁, A₂) werden jeweils mit ca. 1,5 l einer wäßrigen Alkylpyridinhydrochlorid-Lösung befüllt und auf eine Reaktortemperatur von 135 °C eingestellt. Die vorgelegte Katalysatorlösung wird dabei entsprechend der eingestellten Reaktortemperatur entwässert.

In den Reaktor A₁ werden über das Tauchrohr (3) stündlich ein Gemisch aus 0,8 Mol n-Octanol (1 bzw. 11) und 1 Mol Chlorwasserstoff (2) eingeleitet.

Das Reaktionsprodukt gelangt per Überlauf in den Reaktor A₂. Dort wird neben weiteren 0,3 Mol Chlorwasserstoff(2) zusätzlich konzentrierte Salzsäure (36%ig) aus der Leitung (5) zudosiert und das Reaktionsprodukt als Azeotrop über Kopf (4) abgezogen.

Zur Azeotropdestillation wird ein Mengenverhältnis Alkylchlorid : Salzsäure von 1 : 0,7 eingestellt.

Das kondensierte zweiphasige Destillat wird in der Vorlage B getrennt.

Die untere wäßrige Salzsäurephase wird teilweise rückgeführt (5), die übrige Menge wird zur Stripperkolonne (G) gefahren.

Die obere Alkylchloridphase wird über (6) durch alkalische Wäsche (C) und alkalische Trocknung (D) aufgearbeitet und abgetrennt.

Das Abgas, überwiegend Chlorwasserstoff und Stickstoff mit wenig Leichtsiedern und n-Octylchlorid, wird über die Sammelleitung (8) zur Absorption von HCl dem H₂O-Gegenstromwascher (E) und anschließend zur Absorption von n-Octylchlorid dem n-Octanol-Gegenstromwascher (F) zugeführt.

Das so gereinigte Abgas (9) kann einer Entsorgung, z. B. einer Abgasverbrennung, zugeführt werden.

Das mit n-Octylchlorid angereicherte n-Octanol aus dem Gegenstromwascher (F) wird in den Reaktor (A₁) eingespeist.

Die Prozeßabwässer werden auf einen pH-Wert von < 7 eingestellt und zur Entfernung von organischen Bestandteilen der Stripperkolonne (G) zugeführt. Die obere organische Phase des dort anfallenden Azeotrops (überwiegend Olefine, Alkylchloride, Alkohole und wäßrige Salzsäure) wird in den Reaktor (A₁) zurückgeführt und die untere wäßrige Azeotropphase in den oberen Teil der Stripperkolonne gefahren.

Der von organischen Anteilen befreite Sumpf (10) wird einer Abwasseraufbereitungsanlage zugeführt.

Bei dieser Kaskadenfahrweise beträgt der Alkohol-Umsatz im ersten Reaktor ca. 95 %, die Restumsetzung erfolgt in der zweiten Reaktionsstufe.

Produktzusammensetzung und Ausbeute des hergestellten n-Octylchlorids:

| Zusammensetzung laut GC-Analyse | |
|---|---|
| Leichtsieder | 0,7 % |
| n-Octylchlorid | 99 % |
| n-Octanol | 0,2 % |
| Di-n-octylether | 0,1 % |
| APHA-Farbe | ≤ 10 |
| H₂O-Gehalt | ≤ 300 ppm |
| Ausbeute | > 95 % der Theorie |

### Beispiel 2

### Kontinuierliche Herstellung von 1,6-Dichlorhexan (Abb. 1)

Aufgrund der besseren Reaktivität des Diols wird gegenüber dem Beispiel 1 hier nur eine Reaktionsstufe benötigt, um einen > 99%igen Diol-Umsatz zu erzielen.

In den Glasreaktor (A) werden 1,5 1 einer wäßrigen Alkylpyridinhydrochlorid-Lösung vorgelegt, auf 135 °C eingestellt und über das Tauchrohr (3) ein Gemisch aus 0,6 Mol 1,6-Hexandiol, 2 Mol Chlorwasserstoff und konzentrierte Salzsäure im Mengenverhältnis Diol: Salzsäure = 0,6 : 1 zudosiert.

Das dampfförmig ausgetragene Reaktionsprodukt (4) wird kondensiert und die Phasen in der Destillatvorlage B getrennt.

Die weitere Aufarbeitung erfolgt analog Beispiel 1. Der Gegenstromwascher (F) wird mit 1,6-Hexandiol betrieben.

Produktzusammensetzung und Ausbeute des hergestellten 1,6-Dichlorhexans:

| GC-Analyse: | |
|---|---|
| Leichtsieder | < 1 % |
| 1,6-Dichlorhexan | > 98 % |
| 1,6-Hexandiol | 0,1 % |
| Ether | < 0,3 % |
| APHA-Farbe | ≤ 10 |
| H₂O-Gehalt | ≤ 300 ppm |
| Ausbeute | > 95 % der Theorie |

## Patentansprüche

1. Verfahren zur Herstellung von Alkylchloriden mit 6 bis 16 C-Atomen durch Umsetzung der entsprechenden Alkohole mit Chlorwasserstoff,
dadurch gekennzeichnet,
daß man die Reaktion bei einer Temperatur unterhalb der Siedetemperatur des Alkylchlorids durchführt und das Alkylchlorid mit Hilfe von zusätzlich eingespeister konzentrierter Salzsäure über Kopf abdestilliert.

2. Verfahren nach Anspruch 1,
dadurch gekennzeichnet,
daß die Alkylchloride 6 bis 10 C-Atome aufweisen.

3. Verfahren nach Anspruch 1,
dadurch gekennzeichnet,
daß die Reaktionstemperatur 5 bis 100 °C unter der Siedetemperatur des Alkylchlorids liegt.

4. Verfahren nach Anspruch 1,
dadurch gekennzeichnet,
daß es kontinuierlich durchgeführt wird.

## Claims

1. A process for preparing alkyl chlorides having 6 to 16 C atoms by reacting the corresponding alcohols with hydrogen chloride, characterized in that the reaction is carried out at a temperature below the boiling temperature of the alkyl chloride and the alkyl chloride is distilled off overhead using concentrated hydrochloric acid additionally fed in.

2. A process according to claim 1, characterized in that the alkyl chlorides have 6 to 10 C atoms.

3. A process according to claim 1, characterized in that the reaction temperature is 5 to 100°C below the boiling temperature of the alkyl chloride.

4. A process according to claim 1, characterized in that it is carried out continuously.

## Revendications

1. Procédé de préparation de chlorures d'alkyle ayant de 6 à 16 atomes de carbone, par réaction d'alcools correspondants avec de l'acide chlorhydrique,
caractérisé en ce qu'
on effectue la réaction à une température en dessous de la température d'ébullition du chlorure d'alkyle et en ce qu'on sépare par distillation le chlorure d'alkyle à l'aide d'acide chlorhydrique concentré alimenté en supplément sur la tête de colonne.

2. Procédé selon la revendication 1,
caractérisé en ce que
les chlorures d'alkyle possèdent de 6 à 10 atomes de carbone.

3. Procédé selon la revendication 1,
caractérisé en ce que
la température de réaction se situe de 5 à 100°C en dessous de la température d'ébullition du chlorure d'alkyle.

4. Procédé selon la revendication 1,
caractérisé en ce qu'
il est exécuté en continu.
